# EUROPEAN PATENT SPECIFICATION

(11) **EP 3 719 495 B1**
(45) Date of publication and mention of the grant of the patent: **15.03.2023**
(21) Application number: 19382233.5
(22) Date of filing: 01.04.2019
(51) Int. Cl.: G01N 33/18, G01N 29/02

(54) **METHOD AND DEVICE FOR DETERMINING WATER HARDNESS**
VERFAHREN UND VORRICHTUNG ZUR BESTIMMUNG DER WASSERHÄRTE
PROCÉDÉ ET DISPOSITIF DE DÉTERMINATION DE LA DURETÉ DE L'EAU

(43) Date of publication of application: 07.10.2020
(73) Proprietor: BSH Hausgeräte GmbH, 81739 München (DE)
(72) Inventor: Artal Lahoz, Maria Carmen, 50007 Zaragoza (ES); Castro Lapetra, Cristina, Zaragoza (ES); Garcia Romeo, Daniel, 50009 Zaragoza (ES); Gil Trasobares, David, 50009 Zaragoza (ES); Lacadena Muro, Maria Jose, 50008 Zaragoza (ES); Lavega, Rebeca, 26510 Pradejon (ES); Rojo Esteban, Lander, 50009 Zaragoza (ES); Tur Gil, Alejandro Juan, 28029 Madrid (ES)

(56) References cited:
- EP-A1- 2 975 395
- EP-B1- 0 785 427
- CH-A2- 705 686
- FR-A1- 2 708 104

## Description

The present invention relates to a method for determining water hardness and a device for determining water hardness configured to run such a method. The present invention further relates to a home appliance comprising such a device.

Document EP 0 785 427 B1 describes a method for monitoring the scaling power of water flowing through a channel of determined geometry, wherein means for accelerating scale formation on a working electrode are placed in said channel, said means comprising a reference electrode and an auxiliary electrode. The mass of scale on the working electrode is determined by means of a quartz crystal microbalance.

Document EP 2 975 395 A1 describes a method for determining a thickness of a layer of scaling in a household appliance by means of a measuring device. The measuring device is designed to detect a deposition characteristic value by means of a surface acoustic wave.

When using water for a multitude of purposes, particularly in home appliances, minerals solved in the water can precipitate and form deposits on adjacent surfaces, such as deposits of calcium sulfate, barium sulfate or calcium carbonate. Calcium carbonate CaCOs, also known as lime scale and the main compound of such deposits, is formed when calcium hydrogen carbonate Ca(HCO₃)₂ solved in water precipitates due to evaporation of the water or an increasing temperature and/or pH value of the water according to following equilibrium equation:

Ca²⁺_{(aq)} + 2 HCO⁻_{3(aq)} ↔ CaCO₃₍ₛ₎ + CO_{2(g)} + H₂O₍ₗ₎

The amount of minerals solved in water - called water hardness - and the calcium carbonate deposits caused by it can cause a variety of problems in home appliances operating with water, such as flow reduction in pipelines, shortening of component lifetimes, more frequent maintenances, an increase in energy consumption due to deposits on heating elements or weaker cleaning performance of detergents, for example due to interaction with the Ca²⁺ ions.

In order to face problems caused by the lime scale ion exchange resins are used for example, which "soften" water by binding some of the minerals, but also cause glass corrosion by binding Ca²⁺ ions in the glass, particularly when applied too heavily.

Therefore, there is a high interest in determining water hardness as accurately as possible to prevent or remove lime scale. Conventional methods for determining water hardness such as measurement with ion selective electrodes, EDTA titration or conductivity measurement are limited though by their frequent requirement of electrodes and reagent dyes respectively, and also by inaccuracy. Devices based on these methods are limited by their high production costs, frequent maintenance, and a short lifetime.

The present invention therefore deals with the problem of specifying an improved or at least alternative method for determining water hardness which is distinguished in particular by a low requirement for maintenance and reagent dyes, and by a high measurement accuracy. The present invention further deals with the problem of specifying a device for determining water hardness configured to run such a method which is distinguished in particular by low production costs, low maintenance requirement, and a high lifetime.

This problem is solved by the subject matter of the independent patent claims. Preferred embodiments are the subject matter of the dependent patent claims.

Accordingly, the basic idea of the invention is to deposit lime scale on a dedicated surface in a controlled manner via electrochemical reactions, and determine the water hardness by determining the mass of the deposited lime scale via surface acoustic waves.

The method for determining water hardness comprises a step of providing water to be analyzed on a surface. The method further comprises the step of applying a voltage to the water such that deposits are precipitated on the surface, and the step of determining the mass of the deposits by means of a surface acoustic wave sensor. Furthermore, the method comprises determining the water hardness from the determined mass. In this way, water hardness is determined without the need for adding reagent dyes and without having to frequently replace measuring electrodes.

According to the invention, the water hardness is additionally determined in additional steps by means of a radiation source radiating the deposits and a radiation detector detecting radiation emitted from the deposits.

According to an advantageous embodiment, the surface on which the water is provided is part of the surface acoustic wave sensor.

Expediently, the water hardness is determined from a height and/or a surface cover rate of the deposits by means of the radiation detector.

Further preferably, the surface cover rate and, alternatively or additionally, the height are determined by means of the radiation detector detecting the wavelength and/or intensity and, alternatively or additionally, spatial distribution of the, particularly fluorescent, radiation emitted by the deposits. This allows for an especially accurate determination of the water hardness.

Advantageously, the water hardness determined by means of the surface acoustic wave sensor is verified by the water hardness determined by means of the radiation detector. In this way the accuracy of the determined water hardness is increased and less liable to measurement errors.

According to a preferred embodiment, the deposits are removed in an additional step carried out before applying the voltage and/or after determining the water hardness by means of the surface acoustic wave sensor and by means of the radiation detector. In this way existing lime scale is removed, such that boundary conditions are calibrated and a measurement of water hardness is reproducible.

According to a further preferred embodiment, the deposits are removed by applying a voltage to the water with reversed polarity compared to the voltage applied to precipitate the deposits. This allows for a particularly efficient removal.

Expediently, the water hardness is determined from the mass and/or the surface cover rate and/or the height respectively by means of a look-up table or an equation. Via such a process empirically backed data can be put to use.

In an advantageous embodiment, the voltage is applied is generated by using at least two electrodes.

In a further advantageous embodiment, one of the at least two electrodes is a reference electrode. Thereby the applied voltage is chosen more freely and precisely, since the reference electrode potential is well known and stable.

Preferably, the voltage applied such that deposits are precipitated on and, additionally or alternatively, removed from the surface acoustic wave sensor has an absolute value between 0.8 volts and 1.5 volts. In this way a particularly efficient precipitation of lime scale is possible and damage to the at least two electrodes is prevented.

The invention further relates to a device for determining water hardness. The device comprises a surface acoustic wave sensor, at least two electrodes, and an electronic control unit, wherein the electronic control unit is communicating with the at least two electrodes, and with the surface acoustic wave sensor , and the device is configured/programmed to run the method according to the present invention, and runs the method according to the present invention.

The device comprises a radiation source for radiating deposits, wherein the electronic control unit is communicating with the radiation source.

The device comprises a radiation detector for detecting radiation emitted by the deposits, wherein the electronic control unit is communicating with the radiation detector.

Preferably, one of the at least two electrodes of the device is a reference electrode. Thereby the applied voltage is chosen more freely and precisely, since the reference electrode potential is well known and stable.

Furthermore, the invention relates to a home appliance comprising a device according to the present invention. The previously explained advantages of the device according to the invention therefore also transfer to the home appliance. Additionally, less detergent is needed in such a home appliance, thus making it more environmentally friendly.

Further important features and advantages of the invention can be gathered from the sub-claims, from the drawing and from the associated figure description using the drawing.

It goes without saying that the features mentioned above and those still to be explained below can be used not only in the respectively specified combination but also in other combinations or on their own without departing from the scope of the present invention. An example of the invention is illustrated in the drawing and will be explained in more detail in the following description.

In the drawing, schematically:

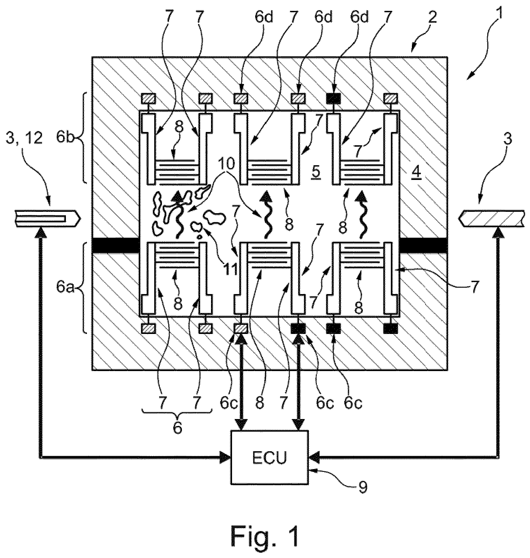

Figure 1 shows a first example of an embodiment of a device for determining water hardness 1 according to the invention in an assembled state in a profile view.

According to the first example of an embodiment shown in figure 1, the device 1 comprises a surface acoustic wave sensor 2 with a base substrate 4. A piezoelectric substrate 5 is arranged on top of the base substrate 4 for guiding a surface acoustic wave 10. Sensor units 6 comprising a transmitter inter-digital transducer 6a for transmitting surface acoustic waves 10 and a receiver inter-digital transducer 6b for receiving surface acoustic waves 10 are arranged on top the piezoelectric substrate 5. The sensor units 6 can be separated, for example by barriers in between them or by grooves in the piezoelectric substrate 5 (not shown in figure 1), or there can be only a single sensor unit 6. Both transducers 6a, 6b comprise comb-shaped, interdigitated finger electrodes 8 attached to bar-type electrodes 7. Additionally, the transmitter inter-digital transducer 6a comprises two transmitter electrodes 6c for supplying an input signal to the corresponding transducer 6a and the receiver inter-digital transducer 6b comprises two receiver electrodes 6d for picking up an output signal from the corresponding transducer 6b.

The device further comprises two electrodes 3 for applying a voltage to water (not shown in figure 1) surrounding the device 1. One of the two electrodes 3 is a reference electrode 12, such as for example a metallic reference electrode, a saturated calomel electrode or a silver chloride electrode. There can also be an electrode (not shown in figure 1) arranged on the surface acoustic wave sensor 2 between the inter-digital transducers 6a, 6b. There can also be a thin guiding film (not shown in figure 1) for guiding surface acoustic waves 10 arranged on the electrode arranged between the inter-digital transducers 6a, 6b on the surface acoustic wave sensor 2 and on the piezoelectric substrate 5. The thin guiding film can comprise SU-8 and, additionally or alternatively, ZnO. The electrodes 3 and also the transmitter and receiver electrodes 6c, 6d of the surface acoustic wave sensor 2 are connected and communicate (not shown for al electrodes 6c, 6d) to an electronic control unit 9.

In operation, the device 1 runs the method for determining water hardness according to the present invention. The method comprises a step of providing water (not shown in figure 1) to be analyzed around the device and on the surface acoustic wave sensor 1. The method further comprises the step of applying a voltage to the water via the electrodes 3 in order to increase the pH value of the water, according to the following equation:

2 H₂O + 2e⁻ → H₂ + 2 OH⁻

or

2 H₃O⁺ + 2 e⁻ → H₂ + 2 H₂O

This way, deposits 11, mainly formed of lime scale, are precipitated on the surface acoustic wave sensor 2, particularly on the piezoelectric substrate 5, or on an electrode arranged between the inter-digital transducers 6a, 6b on the surface acoustic wave sensor 2. The voltage applied can have an absolute value between 0.8 volts and 1.5 volts.

In a subsequent step, the mass of the deposits 11 is determined by means of the surface acoustic wave sensor 2. For this purpose the electronic control unit 9 supplies an input signal to the transmitter inter-digital transducer electrodes 6a via the corresponding transmitter electrodes 6c and picks up an output signal from the receiver inter-digital transducer 6b via the corresponding receiver electrodes 6d. The input signal causes the comb-shaped, interdigitated finger electrodes 8 of the transmitter inter-digital transducer 6a to generate a surface acoustic wave 10 via the inverse piezoelectric effect on the surface of the piezoelectric substrate 5. The surface acoustic wave 10 is then guided by the piezoelectric substrate 5 or by the thin guiding film configured to reduce influences of the water on the surface acoustic waves 10. The surface acoustic wave in turn causes the finger electrodes 8 of the receiver inter-digital transducer 6b to generate the output signal via the piezo electric effect. Barriers or grooves in the piezoelectric substrate 5 in between the sensor units 6 (not shown in figure 1) can help to reduce the interference of different input and output signal pairs of different sensor units 6.

In a next step, the water hardness is determined from the determined mass of the deposits 11. In the example of figure 1, the water hardness is determined from the mass by means of a look-up table or an analytical equation which shows the connection between the mass and the water hardness.

To run the method, the device 1 further comprises a radiation source for radiating deposits and a radiation detector for detecting radiation emitted by the deposits. According to a variant, a height of the deposits on the surface are determined by means of the radiation source radiating the deposits and the radiation detector detecting radiation emitted by the deposits. Alternatively a surface cover rate of the deposits on the surface is determined by means of the radiation source radiating the deposits and the radiation detector detecting radiation emitted or reflected by the deposits.

The radiation source is configured to radiate the deposits with radiation of a wavelength between 240 nm and380. This radiation source is configured particularly by means of a filter system able to configure the radiation source to emit radiation of a wavelength between 240 nm and380 nm.

The height can be determined by means of the radiation detector detecting the wavelength of the radiation emitted by the deposits. Alternatively the height can be determined by means of the radiation detector detecting the intensity of the radiation emitted by the deposits. The height can also be determined by means of the radiation detector detecting the spatial distribution of the radiation emitted by the deposits. Particularly, the radiation emitted by the deposits and detected by the radiation detector can be fluorescent. Correspondingly, the surface cover rate can be detected in the same way as the height.

Then, according to the method for determining water hardness with the example of an embodiment of the device 1 the water hardness is determined from the determined height. Alternatively or additionally, the water hardness is determined from the determined surface cover rate. The water hardness may be determined from the height by means of a look-up table or an equation. The water hardness can also be determined from the surface cover rate by means of a look-up table or an equation. The water hardness is determined by means of the surface acoustic wave sensor and by means of the radiation detector in combination. In this case the water hardness determined by means of the surface acoustic wave sensor can be verified by the water hardness determined by means of the radiation detector, or the other way around.

In a further last step, the deposits 11 are removed from the surface acoustic wave sensor 2 by decreasing the pH value of the water according to following equations:

6 H₂O → O₂ + 4 H₃O⁺ + 4e⁻

or

4 OH⁻ → O₂ + 2 H₂O + 4 e⁻

The deposits are removed by applying a voltage to the water with reversed polarity compared to the voltage applied to precipitate the deposits.

The method according to the present invention is run by the device 1 comprising an electronic control unit, wherein the electronic control unit is communicating with the surface acoustic wave sensor 2 and with the two electrodes 3, and the device 1 is configured/ programmed to run the method according to the present invention.

The device 1 can further be configured/ programmed to automatically remove deposits 11, which were not precipitated due to the method according to the present invention, but due to other causes, particularly before running the method, and particularly due to the usage in a home appliance, or in a water softener system, or in an ionic exchanger or a capacitive deionization system (not shown in figure 1) comprising the device 1.

### LIST OF REFERENCE CHARACTERS/SIGNS

- 1: Device for determining water hardness
- 2: Surface acoustic wave sensor
- 3: Reaction electrodes
- 4: Base substrate
- 5: Piezoelectric substrate
- 6: Sensor unit
- 6a: Transmitter inter-digital transducer
- 6b: Receiver inter-digital transducer
- 6c: Transmitter electrodes
- 6d: Receiver electrodes
- 7: Bar-type electrodes
- 8: Finger electrodes
- 9: Electronic control unit
- 10: Surface acoustic wave
- 11: Deposits
- 12: Reference electrode

## Claims

1. A method for determining water hardness comprising the steps of:
a) Providing water to be analyzed on a surface;
b) Applying a voltage to the water such that deposits (11) are precipitated on the surface; **characterized by** the steps of:
c) Determining the mass of the deposits (11) by means of a surface acoustic wave sensor (2);
d) Determining the water hardness from the mass determined in step c),
wherein in additional steps c2) carried out after step b) the water hardness is additionally determined by means of a radiation source radiating the deposits (11) and a radiation detector detecting radiation emitted from the deposits (11).

2. Method according to claim 1,
**characterized in that**
the surface on which the water is provided in step a) is part of the surface acoustic wave sensor (2) used in step c).

3. Method according to claim 1 or 2,
**characterized in that**
in steps c2) the water hardness is determined from a height and/or a surface cover rate of the deposits (11) by means of the radiation detector.

4. The method according to claim 3,
**characterized in that**
the surface cover rate and/or the height are determined by means of the radiation detector detecting the wavelength and/or intensity and/or spatial distribution of the, particularly fluorescent, radiation emitted by the deposits (11).

5. Method according to any of claims 1 to 4,
**characterized in that**
the water hardness determined in step d) is verified by the water hardness determined in steps c2).

6. The method according to any of claims 1 to 5,
**characterized in that**
in at least one additional step x) carried out before step b) and/or after step d) and/or after steps c2) the deposits (11) are removed.

7. The method according to any of claims 1 to 6,
**characterized in that**
in step x) the deposits (11) are removed by applying a voltage to the water with reversed polarity compared to the voltage applied in step b).

8. The method according to any of claims 1 to 7,
**characterized in that**
in step d) and/or steps c2) the water hardness is determined from the mass and/or the surface cover rate and/or the height respectively by means of a look-up table or an equation.

9. The method according to any of claims 1 to 8,
**characterized in that**
the voltage applied in step b) and/or step x) is generated by at least two electrodes (3).

10. The method according to claim 9,
**characterized in that**
one of the at least two electrodes (3) is a reference electrode (12).

11. The method according to any of claims 1 to 10,
**characterized in that**
the voltage applied in step b) and/or step x) has an absolute value between 0.8 volts and 1.5 volts.

12. A device (1) for determining water hardness comprising
- a surface acoustic wave sensor (2), **characterized in that** the device (1) comprises
- at least two electrodes (3), and
- an electronic control unit (9), wherein the electronic control unit (9) is communicating with the surface acoustic wave sensor (2) and wherein the device (1) is configured/ programmed to run the method according to any of claims 1 to 11, and wherein the device (1) comprises a radiation source for radiating deposits (11) and a radiation detector for detecting radiation emitted by the deposits (11).

13. The device according to claim 12,
**characterized in that**
one of the at least two electrodes (3) is a reference electrode (12).

14. A home appliance comprising a device (1) according to claims 12 to 13.

## Patentansprüche

1. Verfahren zum Bestimmen der Wasserhärte mit folgenden Schritten:
a) Bereitstellen von zu analysierendem Wasser auf einer Oberfläche,
b) Anlegen einer Spannung an das Wasser, so dass sich Ablagerungen (11) an der Oberfläche absetzen, **gekennzeichnet durch** folgende Schritte:
c) Bestimmen der Masse der Ablagerungen (11) mithilfe eines Oberflächenwellensensors (2),
d) Bestimmen der Wasserhärte anhand der in Schritt c) bestimmten Masse,
wobei die Wasserhärte bei zusätzlichen Schritten c2), die nach Schritt b) durchgeführt werden, zusätzlich mithilfe einer Strahlungsquelle, die die Ablagerungen (11) bestrahlt, und eines Strahlungsdetektors bestimmt wird, der von den Ablagerungen (11) emittierte Strahlung erfasst.

2. Verfahren nach Anspruch 1,
**dadurch gekennzeichnet, dass**
die Oberfläche, auf der das Wasser in Schritt a) bereitgestellt wird, zu dem in Schritt c) benutzten Oberflächenwellensensor (2) gehört.

3. Verfahren nach Anspruch 1 oder 2,
**dadurch gekennzeichnet, dass**
die Wasserhärte bei den Schritten c2) anhand einer Höhe und/oder einer Oberflächenabdeckung der Ablagerungen (11) mithilfe des Strahlungsdetektors bestimmt wird.

4. Verfahren nach Anspruch 3,
**dadurch gekennzeichnet, dass**
die Oberflächenabdeckung und/oder die Höhe mithilfe des Strahlungsdetektors bestimmt wird, der die Wellenlänge und/oder die Intensität und/oder die räumliche Verteilung der von den Ablagerungen (11) emittierten, insbesondere fluoreszierenden Strahlung erfasst.

5. Verfahren nach einem der Ansprüche 1 bis 4,
**dadurch gekennzeichnet, dass**
die in Schritt d) bestimmte Wasserhärte anhand der in den Schritten c2) bestimmten Wasserhärte überprüft wird.

6. Verfahren nach einem der Ansprüche 1 bis 5,
**dadurch gekennzeichnet, dass**
in mindestens einem zusätzlichen Schritt x), der vor dem Schritt b) und/oder nach dem Schritt d) und/oder nach den Schritten c2) durchgeführt wird, die Ablagerungen (11) entfernt werden.

7. Verfahren nach einem der Ansprüche 1 bis 6,
**dadurch gekennzeichnet, dass**
in Schritt x) durch Anlegen einer Spannung mit im Vergleich zu der in Schritt b) angelegten Spannung umgekehrter Polarität an das Wasser die Ablagerungen (11) entfernt werden.

8. Verfahren nach einem der Ansprüche 1 bis 7,
**dadurch gekennzeichnet, dass**
in Schritt d) und/oder den Schritten c2) die Wasserhärte mithilfe einer Nachschlagetabelle oder einer Gleichung anhand der Masse beziehungsweise der Oberflächenabdeckung beziehungsweise der Höhe bestimmt wird.

9. Verfahren nach einem der Ansprüche 1 bis 8,
**dadurch gekennzeichnet, dass**
die in Schritt b) und/oder in Schritt x) angelegte Spannung von mindestens zwei Elektroden (3) erzeugt wird.

10. Verfahren nach Anspruch 9,
**dadurch gekennzeichnet, dass**
es sich bei einer der mindestens zwei Elektroden (3) um eine Referenzelektrode (12) handelt.

11. Verfahren nach einem der Ansprüche 1 bis 10,
**dadurch gekennzeichnet, dass**
die in Schritt b) und/oder in Schritt x) angelegte Spannung einen Absolutwert aufweist, der zwischen 0,8 Volt und 1,5 Volt liegt.

12. Vorrichtung (1) zum Bestimmen der Wasserhärte, die Folgendes umfasst:
- einen Oberflächenwellensensor (2), **dadurch gekennzeichnet, dass** die Vorrichtung (1) Folgendes umfasst:
- mindestens zwei Elektroden (3) und
- ein elektronisches Steuergerät (9), wobei das elektronische Steuergerät (9) mit dem Oberflächenwellensensor (2) kommuniziert und die Vorrichtung (1) so konfiguriert/programmiert ist, dass sie das Verfahren nach einem der Ansprüche 1 bis 11 ausführt, und die Vorrichtung (1) eine Strahlungsquelle zum Bestrahlen von Ablagerungen (11) und einen Strahlungsdetektor zum Erfassen von von den Ablagerungen (11) emittierter Strahlung umfasst.

13. Vorrichtung nach Anspruch 12,
**dadurch gekennzeichnet, dass**
es sich bei einer der mindestens zwei Elektroden (3) um eine Referenzelektrode (12) handelt.

14. Haushaltsgerät mit einer Vorrichtung (1) nach Anspruch 12 bis 13.

## Revendications

1. Procédé de détermination de la dureté de l'eau comprenant les étapes de :
a) fourniture d'eau à analyser sur une surface ;
b) application d'une tension à l'eau de sorte que des dépôts (11) soient précipités sur la surface ; **caractérisé par** les étapes de :
c) détermination de la masse des dépôts (11) au moyen d'un capteur à ondes acoustiques de surface (2) ;
d) détermination de la dureté de l'eau au départ de la masse déterminée à l'étape c),
dans lequel lors d'étapes supplémentaires c2) effectuées après l'étape b), la dureté de l'eau est en outre déterminée au moyen d'une source de rayonnement qui irradie les dépôts (11) et d'un détecteur de rayonnement détectant le rayonnement émis par les dépôts (11).

2. Procédé selon la revendication 1, **caractérisé en ce que** la surface sur laquelle l'eau est fournie à l'étape a) fait partie du capteur à ondes acoustiques de surface (2) utilisé à l'étape c).

3. Procédé selon la revendication 1 ou 2, **caractérisé en ce qu'**aux étapes c2), la dureté de l'eau est déterminée au départ d'une hauteur et/ou d'un taux de couverture de surface des dépôts (11) au moyen du détecteur de rayonnement.

4. Procédé selon la revendication 3, **caractérisé en ce que** le taux de couverture de surface et/ou la hauteur sont déterminés au moyen du détecteur de rayonnement détectant la longueur d'ondes et/ou l'intensité et/ou la distribution spatiale du rayonnement, en particulier fluorescent, émis par les dépôts (11).

5. Procédé selon l'une quelconque des revendications 1 à 4, **caractérisé en ce que** la dureté de l'eau déterminée à l'étape d) est vérifiée par la dureté de l'eau déterminée aux étapes c2).

6. Procédé selon l'une quelconque des revendications 1 à 5, **caractérisé en ce que** dans au moins une étape supplémentaire x) effectuée avant l'étape b) et/ou après l'étape d) et/ou après les étapes c2), les dépôts (11) sont enlevés.

7. Procédé selon l'une quelconque des revendications 1 à 6, **caractérisé en ce qu'**à l'étape x), les dépôts (11) sont enlevés en appliquant à l'eau une tension de polarité inversée par rapport à la tension appliquée à l'étape b).

8. Procédé selon l'une quelconque des revendications 1 à 7, **caractérisé en ce qu'**à l'étape d) et/ou aux étapes c2), la dureté de l'eau est déterminée au départ de la masse et/ou du taux de couverture de surface et/ou de la hauteur respectivement au moyen d'une table à consulter ou d'une équation.

9. Procédé selon l'une quelconque des revendications 1 à 8, **caractérisé en ce que** la tension appliquée à l'étape b) et/ou à l'étape x) est générée par au moins deux électrodes (3).

10. Procédé selon la revendication 9, **caractérisé en ce que** l'une des au moins deux électrodes (3) est une électrode de référence (12).

11. Procédé selon l'une quelconque des revendications 1 à 10, **caractérisé en ce que** la tension appliquée à l'étape b) et/ou à l'étape x) possède une valeur absolue entre 0,8 volt et 1,5 volt.

12. Dispositif (1) de détermination de la dureté de l'eau comprenant
- un capteur à ondes acoustiques de surface (2), **caractérisé en ce que** le dispositif (1) comprend
- au moins deux électrodes (3), et
- une unité de commande électronique (9), dans lequel l'unité de commande électronique (9) communique avec le capteur à ondes acoustiques de surface (2) et dans lequel le dispositif (1) est configuré/ programmé pour exécuter le procédé selon l'une quelconque des revendications 1 à 11, et dans lequel le dispositif (1) comprend une source de rayonnement pour irradier les dépôts (11) et un détecteur de rayonnement pour détecter le rayonnement émis par les dépôts (11).

13. Dispositif selon la revendication 12, **caractérisé en ce que** l'une des au moins deux électrodes (3) est une électrode de référence (12).

14. Appareil ménager comprenant un dispositif (1) selon les revendications 12 à 13.
